# EUROPÄISCHE PATENTSCHRIFT

(11) **EP 3 708 100 B1**
(45) Veröffentlichungstag und Bekanntmachung des Hinweises auf die Patenterteilung: **02.10.2024**
(21) Anmeldenummer: 20163675.0
(22) Anmeldetag: 16.08.2017
(51) Int. Cl.: A61B 18/02, A61B 5/0215, A61B 5/20, A61B 18/00

(54) **MEDIZINISCHES GERÄT ZUR DENERVIERUNG RENALER PERIVASKULÄRER NERVEN**
MEDICAL DEVICE FOR DENERVATION OF RENAL PERIVASCULAR NERVES
APPAREIL MÉDICAL PERMETTANT DE DÉNERVER LES NERFS PÉRIVASCULAIRES RÉNAUX

(30) Priorität: 18.08.2016 DE 102016115387
(43) Veröffentlichungstag der Anmeldung: 16.09.2020
(62) Teilanmeldung aus: 17772600.7
(73) Patentinhaber: Cryovasc GmbH, 10119 Berlin (DE)
(72) Erfinder: Keweloh, Boris, 10119 Berlin (DE)
(74) Vertreter: Stütz, Jan

(56) Entgegenhaltungen:
- DE-U1- 202016 104 549
- US-A1- 2009 299 355
- US-A1- 2011 264 086
- US-A1- 2012 029 511
- US-A1- 2013 131 743
- US-A1- 2014 249 520
- US-A1- 2015 066 007

## Beschreibung

Die Erfindung betrifft ein medizinisches Gerät zur Denervierung renaler perivaskulärer Nerven.

Nach dem Stand der Technik werden derartige Geräte eingesetzt, um Patienten mit Hypertonie bzw. Bluthochdruck zu behandeln.

In den meisten Fällen lässt sich der Bluthochdruck durch eine angepasste Ernährung wie etwa die Reduzierung des Salz- und Alkoholkonsums sowie eine Steigerung der sportlichen Betätigung beseitigen oder reduzieren. Auch die Reduzierung eines Übergewichts und die Vermeidung von Stress können den Blutdruck senken. Bei Patienten mit höherem Blutdruck kommen pharmazeutische Präparate zum Einsatz, welche in der richtigen Dosierung bzw. bei richtiger Einstellung des Patienten dauerhaft den Blutdruck in akzeptablen Grenzen halten.

Es gibt aber auch Patienten, bei denen eine nachhaltige Blutdrucksenkung mit langfristig anwendbaren Präparaten nicht möglich ist.

Diese Patienten leiden meist an einer besonders stark ausgeprägten Hyperaktivität des sympathischen Nervensystems.

Es hat sich gezeigt, dass trotz einer bestehenden Multikausalität der Hypertonie, die Aktivität der renalen perivaskulären Nerven eine große Rolle spielt.

Insbesondere Patienten bei denen es nicht möglich ist, mit Hilfe von pharmazeutischen Präparaten den Blutdruck dauerhaft auf ein akzeptables Niveau einzustellen, werden aus diesem Grund bereits mit medizinischen Geräten zur Denervierung der renalen perivaskulären Nerven behandelt.

Bislang hat die Denervierung der renalen perivaskulären Nerven nicht immer den gewünschten Erfolg.

Bei vielen Patienten fällt die Blutdrucksenkung nach einer Zeit von etwa 6 Monaten nach der Behandlung so gering aus, dass das Risiko eines invasiven Eingriffes in einem schlechten Verhältnis zu möglichen Heilungschancen steht.

Das Dokument US 2014/249520 A1 offenbart Systeme, Geräte und Verfahren zur Erleichterung der Überwachung und/oder Überprüfung des Ergebnisses von Verfahren, wie z. B. Nerven-Denervierungs- und/oder Nerven-Stimulationsverfahren. Dabei wird der Zustand des Gewebes nach jedem Verfahren, einschließlich jedes Ablationszyklus bzw. einer Reihe von Ablationszyklen, beurteilt. Das Dokument US 2014/249520 A1 offenbart auch ein Gerät zur Denervierung renaler Nerven mittels eines Kryoballons umfassend einen Drucksensor auf der Außenseite des Kryoballons.

Dokument US 2015/066007 A1 beschreibt Neuromodulationskatheter, die neurale Signale vor und/oder nach einer Neuromodulation aufzeichnen, und einen Digitalisierer, der die aufgezeichneten neuralen Signale digitalisiert und die digitalisierten neuralen Signale an ein extrakorporales Gerät überträgt. Dabei werden auch Vorrichtungen zur Neuromodulation offenbart, wie beispielsweise ein Kryoballon zur Ablation renaler Nerven, wobei die Nerven zirkulär abladiert werden. Auch ein Drucksensor an dem als Kryoballon ausgebildeten Katheter ist offenbart.

Das Dokument US 2013131743 A1 bezieht sich auf Vorrichtungen, Systeme und Verfahren zur intraoperativen Überwachung der Nervenaktivität, um die Auswirkungen einer elektrisch und/oder thermisch induzierten Neuromodulation zu bestimmen. Insbesondere beschreibt Dokument US 2013131743 A1 einen Führungsdraht umfassend zwei voneinander beabstandete Schleifen, in denen Elektroden zur Messung der Nervenstimulation angeordnet sind. Diese Vorrichtungen können auch Anwendungen zur Neuromodulation enthalten. In diesem Zusammenhang wird auch die Ablation renaler Nerven mittels eines Kryoballons im Allgemeinen beschrieben.

Dokument US 2011/264086 A1 beschreibt Vorrichtungen zur Denervierung an einer Nierenarterie umfassend eine längliche Führungsschiene mit einem spiralförmigen Abschnitt. Die Vorrichtung umfasst ein Behandlungselement zur Durchführung einer Denervierungstherapie, u.a. einer Kryoablation. Das Behandlungselement ist auf einem Behandlungskatheter aufgebracht, der durch Aufblasen eines Ballons eine Spirale annimmt, um den Kontakt zwischen zumindest Teilen des Behandlungsabschnitts und der Innenwand der Nierenarterie zu erleichtern.

Die Ursache für die eingeschränkte Wirksamkeit von Nierenablationsverfahren aus dem Stand der Technik war bislang nicht bekannt.

Die Aufgabe der Erfindung besteht darin, ein Gerät zu Verfügung zu stellen, welche die aus dem Stand der Technik bekannten Nachteile zumindest reduziert und eine nachhaltigere Senkung des Blutdrucks durch die renale Denervierung ermöglicht.

Diese Aufgabe wird mit einem medizinischen Gerät nach Anspruch 1 gelöst. Die Unteransprüche stellen bevorzugte Ausführungsformen der Erfindung dar.

Das erfindungsgemäße medizinische Gerät zur Denervierung renaler perivaskulärer Nerven verfügt dabei über einem Katheter mit einem flexiblen Schaft zum Einführen in die renale Arterie eines Patienten.

Der Schaft hat dabei ein inneres Lumen, welches zum Zuführen eines Kältemittels geeignet ist. Am distalen Ende des Schaftes weist das medizinische Gerät einen Kryoballon auf. Dieser Kryoballon steht mit dem Lumen des Schaftes in Verbindung und wird über den Schaft mit einem Kältemittel versorgt. Durch die Verdampfung des Kältemittels in dem Ballon wird auf der Oberfläche des Ballons eine Temperatur vorzugsweise zwischen - 30 und -80 °C erzeugt, mit deren Hilfe die renalen perivaskulären Nerven abladiert werden.

Auf der Oberfläche des Ballons ist wenigstens eine bipolare Elektrode angeordnet und mit einer Stromquelle zur Abgabe eines elektrischen Impulses oder einer Abfolge von Impulsen oder einer Frequenz zur Stimulation der perivaskulären Nerven verbunden.

Diese Anregung der perivaskulären Nerven wird für einen Feedbackloop verwendet, um den Behandlungserfolg zu überprüfen.

Dabei ist das erfindungsgemäße medizinische Gerät dadurch gekennzeichnet, dass der Kryoballon dazu ausgebildet ist, im Anwendungsfall die Wand der renalen Arterie (in Blutströmungsrichtung) auf einer Länge von mehr als 2,5 cm, vorzugsweise mindestens 3 cm und besonders bevorzugt wenigstens 3,5 cm zu berühren und auf einer Länge von mindestens 2,5 cm, vorzugsweise mindestens 3 cm und besonders bevorzugt wenigstens 3,5 cm (in Blutströmungsrichtung) durchgehend und gleichzeitig zu abladieren.

Im expandierten Zustand hat der Kryoballon einen Durchmesser von zwischen 3 und 7 mm vorzugsweise zwischen 4 und 6 mm.

Aus dem medizinischen Prinzip heraus, das geringstmögliche Gewebevolumen zu abladieren, um einen medizinischen Erfolg (hier die Ablation und Unterbrechung der Reizleitung der renalen perivaskulären Nerven) zu erzielen, ist das Vorsehen von einer derart großen Ablationsoberfläche bei Geräten aus dem Stand der Technik nicht erwünscht gewesen. Vor allem, da das Risiko einer Gefäßverengung, einer Blutgerinnselauflagerung und einer Gefäßperforation kritisch betrachtet werden muss.

Darüber hinaus wurde eine Unterbrechung der Reizleitung auch schon bei einer Ablation von bestimmten Bereichen in der renalen Arterie erzielt, sodass großflächige Ablationen gezielt vermieden wurden.

Umso überraschender war das Ergebnis, dass mit mehreren ausführlichen Kälteablationen der renalen perivaskulären Nerven auf möglichst der gesamten Länge der renalen Arterie und bevorzugt der gesamten Zirkumferenz eine nachhaltige Blutdrucksenkung bei den behandelten Patienten erzielt werden kann.

Das erfindungsgemäße medizinische Gerät ist dementsprechend von den geometrischen Abmessungen dazu ausgebildet, die Nierenarterie im Großteil ihrer Länge und in ihrer gesamten Zirkumferenz zu abladieren.

Außerdem verwendet das erfindungsgemäße medizinische Gerät Kälte (Kryoenergie) und nicht Radiofrequenzenergie (Hitze). Kälteablationen reißen die Gefäßinnenwand seltener ein, es kommt seltener zur Auflagerung von Blutgerinnseln und die Gefahr von Perforationen des Gefäßes ist geringer.

Im Anwendungsfall ist der Katheter, auf dem der Kryoballon angeordnet ist, mit einer Kältemittelquelle und einer Steuerung für die Kältemittelquelle verbunden.

Erfindungsgemäß wird dabei die Kältemittelquelle bzw. die Zufuhr des Kältemittels in den Kryoballon in Abhängigkeit von einem Blutdruckwert des Patienten während der Behandlung gesteuert.

Dabei verfügt der Katheter über einen Drucksensor. Hierdurch kann während der Denervierung eine direkte (invasive) Blutdruckmessung durchgeführt werden. Der Blutdruckwert wird dabei erfasst und der Steuerung zugeführt, welche in Abhängigkeit von diesem Wert die Kältemittelquelle bzw. die Zufuhr des Kältemittels zum Kryoballon steuert.

Nicht Teil der gemäß Anspruch 1 geschützten Erfindung ist vorgesehen, dass die Steuerung mit einer Blutdruckmanschette verbunden ist und der aktuelle Blutdruck dementsprechend indirekt gemessen wird. Aber auch hier findet eine Steuerung der Kältemittelquelle bzw. die Zufuhr des Kältemittels zum Kryoballon in Abhängigkeit von dem gemessenen Blutdruck statt.

Eine invasive Blutdruckmessung bietet jedoch noch weitere Vorteile: Erfindungsgemäß wird mit dem Sensor die Druckanstiegsgeschwindigkeit, also delta mmHg/ s gemessen. Besonders bevorzugt erfolgt dies innerhalb des Anstieges eines Schlages, als Maß der Herzkraft und des Blutdrucks. Hierdurch ist es möglich, viel früher und sicherer zu diagnostizieren, ob die renalen perivaskulären Nerven schon ausreichend abladiert sind oder noch stimuliert werden können.

Nach einer weiteren Ausgestaltung der Erfindung ist auch die Stromquelle zur Abgabe eines elektrischen Impulses oder einer elektrischen Frequenz über wenigstens eine Elektrode mit der Steuerung verbunden. Die Steuerung leitet dann zunächst die Abgabe eines oder einer Reihe von Impulsen oder eine Frequenz über wenigsten eine Elektrode ein. Dieser Vorgang kann auch als Anregungsphase bezeichnet werden.

Daraufhin prüft die Steuerung mit Hilfe des gemessenen Blutdrucks oder eines korrelierenden Wertes, ob eine Blutdruckerhöhung innerhalb eines vorgegebenen Zeitintervalls und oberhalb eines vorgegebenen Grenzwertes erfolgt ist.

Ist dies der Fall, dann wird eine Ablationsphase durch Ansteuerung der Kältemittelquelle eingeleitet oder eine Ablationsphase wird zumindest für eine vorherbestimmte Zeit fortgesetzt oder aber wiederholt.

Vorzugsweise wird in diesem Zusammenhang der Blutdruck oder ein korrelierender Wert, welcher kurz vor oder während der Anregung der perivaskulären Nerven mit Hilfe elektrischer Impulse gemessen wurde, mit dem Wert des Blutdrucks nach einer Zeit von zwischen 15 Sekunden und 5 Minuten, weiter bevorzugt nach einer Zeit von zwischen 2 und 4 Minuten nach Beginn der Anregungsphase verglichen.

Die Anregung der perivaskulären Nerven erfolgt z. B. mit einer Frequenz von 20HZ, einer Spannung von 15 Volt (Amplitude) und einer Länge von wenigstens 10ms.

Der Grenzwert der minimalen Druckerhöhung, bei der eine Ablationsphase eingeleitet oder aufrechterhalten wird, beträgt vorzugsweise 5 mmHG.

Die Ablationphase beträgt vorzugsweise zwischen 2 und 3 Minuten, wobei der Ballon an der Oberflächen bevorzugt eine Temperatur von unter -50 °C aufweist.

Nach einer bevorzugten Ausführungsform wird die Ablationsphase beendet, wenn trotz einer Anregung, welche auch während der Ablationsphase stattfindet, der Blutdruck nicht über einen vor Beginn der Anregungsphase gemessenen Wert hinausgeht.

Nach Beendigung einer Ablationsphase ist die Steuerung vorzugsweise dazu ausgebildet, nach einer Zeit von zwischen 5 und 20 Minuten eine weitere Anregungs- und ggf. Ablationsphase zu starten.

Besonders bevorzugt startet die Steuerung zwischen 5 und 10 Minuten nach Beendigung einer Ablationsphase eine weitere Anregungsphase. Wird bei Anregung aber keine Blutdruckerhöhung um einen vorgegebenen Grenzwert erfasst, so startet die Steuerung nach erneut 5 bis 10 Minuten eine weitere Anregungsphase. Wird hierbei oder gemäß einer Abwandlung der Ausführungsform bei einer weiteren Anregungsphase weiterhin keine Blutdruckerhöhung festgestellt, so wird die Behandlung beendet. Ansonsten startet der Zyklus von vorne.

Gemäß einer weiteren Ausführungsform ist vorgesehen, dass die Elektroden ImpedanzMessungen vornehmen. Nach einer bevorzugten Ausführungsform wird die Impedanz-Messung von der Steuerung dazu verwendet, einen Wert für die Läsionstiefe zu ermitteln. Vorzugsweise ist zudem vorgesehen, dass in der Steuerung ein Grenzwert für die Läsionstiefe abgelegt ist. Entsprechend wird der Behandlungszyklus abgebrochen, wenn die mit Hilfe der Elektroden durchgeführte Impedanz-Messung einen Wert ergibt, welcher das Überschreiten einer zulässigen hiermit zusammenhängenden Läsionstiefe kennzeichnet.

Nach einer weiteren Ausführungsform ist der Kälteballon so ausgebildet, dass er einen an der Außenseite in Längsrichtung verlaufenden bevorzugt kälteisolierten Kanal aufweist, welcher sich radial bevorzugt zwischen 10 und 40 % seiner Zirkumferenz und weiter bevorzugt zwischen 25 und 35% seiner Zirkumferenz erstreckt. Der Kanal hat bevorzugt die Form eines Zylindersektors.

Durch diesen Kanal kann während der 2-3 Minuten andauernden Ablation Blut zur Niere strömen. Dies verhindert etwaige Schäden des Nierengewebes durch eine wiederholte Blutleere.

Gemäß einer Ausführungsform weist der Kryoballon an der Stelle des zu bildenden Kanals einen Streifen nicht oder wenig dehnbaren Materials auf. Dieser Streifen, welcher auf den Katheter oder auch auf den Kryoballon geklebt sein kann oder teilweise das ursprüngliche dehnbare Material des Kryoballons ersetzt, verhindert eine Ausdehnung des Kryoballons an dieser Stelle, wodurch ein Kanal im Querschnitt der Arterie freigehalten wird, damit es zu einer Strömung von Blut zur Niere auch während der Behandlung kommt.

Wenn der Zylindersektor einen Radius von mehr als 180° aufspannt, kann gewährleistet werden, dass die gesamte Zirkumferenz der Gefäßwand mit dem Kryoballon bei einer zweimaligen Anwendung in Kontakt kommt, wenn der Kryoballon während der Behandlung wenigstens einmal um die Längsachse um einen Minimalwinkel gedreht wird, welcher größer, als der Winkel ist, welchen der Zylindersektor des Kanals 8 aufspannt.

Gemäß einer anderen Ausführungsform ist vorgesehen, dass der Kryoballon zwar zylinderförmig ausgebildet ist, jedoch einen Bypass-Kanal mit anderen Worten einen Tunnel durch den Ballon aufweist, welcher einen Blutfluss zur Niere auch während der Behandlung ermöglicht.

Weitere Details sind aus der Figurenbeschreibung zu entnehmen, welche bevorzugte Ausführungsformen der Erfindung darstellen.

Dabei zeigen:
Fig. 1 das erfindungsgemäße medizinische Gerät mit Kältemittelquelle und Steuereinheit,
Fig. 2 den Katheter in einer ersten Ausführungsform in einer Seitenansicht,
Fig. 3 den Katheter aus Figur 2 in einer Querschnittsdarstellung der Ebene A-A,
Fig. 4 eine Querschnittsdarstellung einer zweiten Ausführungsform des Katheters,
Fig. 5 eine Querschnittsdarstellung einer dritten Ausführungsform des Katheters.

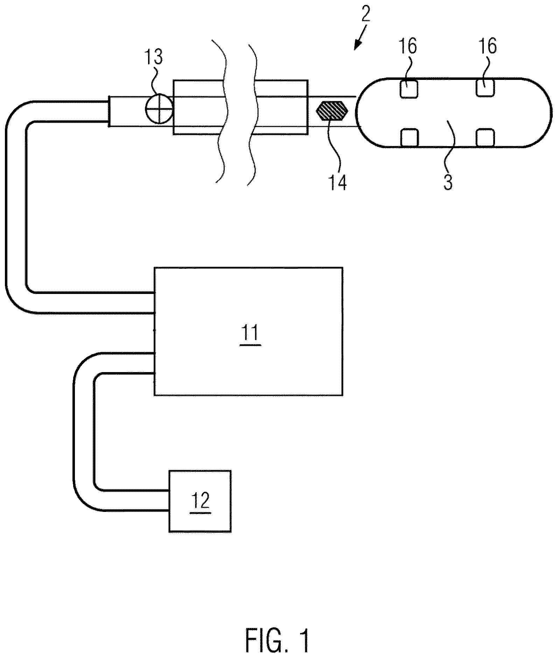

Figur 1 zeigt das erfindungsgemäße medizinische Gerät 1. Das Gerät umfasst dabei einen Katheter 2 mit einer flexiblen Mantelfläche, welcher zum Einführen in die Arterie eines Menschen und speziell in die renale Arterie ausgebildet ist.

Am distalen Ende ist der Katheter 2 expandierbar ausgebildet und bildet im Anwendungsfall einen Kryoballon 3 zum Abladieren von Körpergewebe.

Am Schaft des Katheters ist eine Vorrichtung zur Messung des Blutdrucks 14 angeordnet. In der Nähe des proximalen Endes des Katheters 2 ist eine Markierung 13 angebracht, mit deren Hilfe ein Drehen des Katheters um seine Längsachse während der Behandlung festgestellt werden kann.

Am proximalen Ende des Katheters sind eine elektrische Verbindung und eine Verbindung für ein Kältemittel vorgesehen, welche den Katheter 2 mit einer Steuerung 11 verbindet. Die Steuerung selbst ist zudem mit einer Quelle für ein Kältemittel 12 verbunden und steuert im Anwendungsfall die Zuführung von Kältemittel in den am distalen Ende des Katheters 2 angeordneten Kryoballon 3.

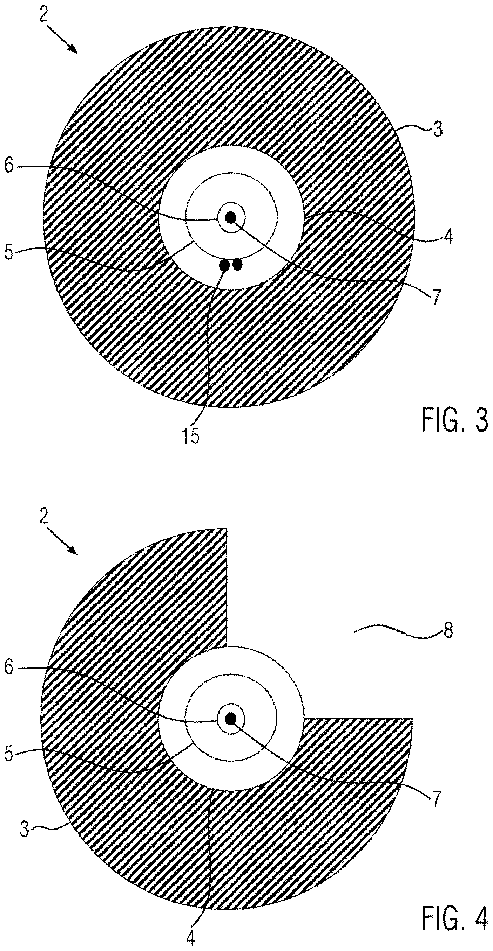

Über elektrische Leitungen 15 (siehe Figur 3) ist die Steuerung 11 zudem mit dem Drucksensor 14 zur Bestimmung des Blutdruckes verbunden. Weitere elektrische Leitungen verbinden vier auf dem Kryoballon 3 angeordnete Elektroden 16 mit der Steuerung 11.

Im Anwendungsfall stimuliert wenigstens eine der Elektroden 16 die renalen perivaskulären Nerven eines Patienten. Die Steuerung 11 stellt fest, ob auf die Stimulierung durch die Elektrode(n) 16 eine Blutdruckerhöhung oder die Erhöhung eines mit dem Blutdruck korrelierenden Wertes innerhalb eines vorherbestimmten Zeitintervalls und oberhalb eines vorgegebenen Grenzwertes stattgefunden hat.

Ist dies der Fall, so leitet die Steuerung 11 Kältemittel aus der Kältemittelquelle 12 über den Katheter 2 in den Kryoballon 3 und bewirkt eine Ablation der renalen perivaslukären Nerven. Eine Markierung 13 ist auf dem Katheter vorgesehen, welche gestattet, die Drehung des Katheters um seine Längsachse festzustellen und zu prüfen, ob eine gewünschte Mindestdrehung bei der Behandlung durchgeführt wurde.

Dies ist vor allem bei Ausführungsformen sinnvoll, bei denen der Kryoballon 3 so ausgebildet ist, dass er im Querschnitt und im Behandlungsfalle nicht die gesamte Zirkumferenz der arteriellen Gefäßwand berührt.

In diesem Falle ist es für die Behandlung notwendig, die Ablation der renalen perivaskulären Nerven in wenigstens zwei und bevorzugt genau zwei Behandlungsschritten durchzuführen. Der Katheter muss während der Behandlung dann wenigstens einmal um einen vorgegebenen Mindestwinkel um die Längsachse des Katheters 2 verdreht werden.

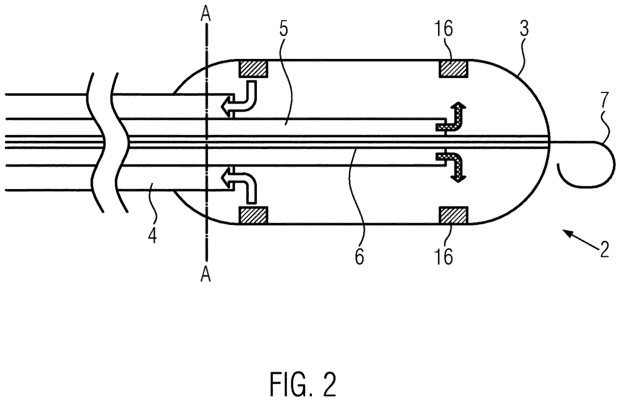

Figur 2 zeigt einen Längsschnitt durch den Katheter 2 in einer ersten Ausführungsform mit symmetrischem Querschnitt. Der Katheter verfügt dabei über 3 Lumen. Das innerste Lumen 6 ist zur Aufnahme eines J-wires ausgebildet und entsprechend dimensioniert. Im Behandlungsfalle hilft der J-wire, den Katheter in die renale Arterie eines Patienten einzuführen.

Sobald der Katheter 2 an seinem Bestimmungsort in der renalen Arterie des Patienten angelangt ist, wird über das äußerste Lumen 4 Druckluft in den Kryoballon 3 geleitet, wodurch dieser sich entfaltet und an die Gefäßwand der Arterie anlegt. Mittels der Elektroden 16 wird dann eine Stimulation der renalen perivaskulären Nerven bewirkt, was normalerweise zu einer Blutdruckerhöhung führt.

Wird eine Blutdruckerhöhung festgestellt, tritt flüssiges Kältemittel (bevorzugt N₂O) über das distale Ende des Lumens 5 in den Kryoballon 3 ein und verdampft. Durch die Verdampfung wird Kälte erzeugt, welche über die Wandung des Kryoballons 3 an das Gewebe abgegeben wird und durch Ablation zu einer Denervierung führt. Die expandierten Gase werden dann über das äußerste Lumen 4 zum proximalen Ende des Katheters 2 geleitet und an die Narkosegasabsaugung des Katheterlabores/Operationssaales abgegeben.

Anders als in dieser Ausführungsform kann auch vorgesehen sein, dass das flüssige Kältemittel über Löcher in den Kryoballon 3 eintritt, welche über die Länge des Kryoballons verteilt in der Wandung des Katheters 2 angeordnet sind.

Figur 3 zeigt einen Querschnitt der in Figur 2 dargestellten Ausführungsform des Katheters entlang der Schnittlinie A-A. Von innen nach außen zeigt Figur 3 den J-wire 7, das Lumen 6 zur Führung des J-wires, das Lumen 5 zur Führung des flüssigen Kältemittels, das Lumen 4 zur ersten Befüllung des Kryoballons 3 mit Druckluft und zur anschließenden Abführung des gasförmigen Kältemittels.

Die mit dem Drucksensor 14 und den Elektroden 16 verbundenen elektrischen Leitungen 15 verlaufen in dieser Ausführungsform im Lumen für die Abführung des dampfförmigen Kältemittels (N₂O).

Figur 4 stellt eine zweite Ausführungsform des Katheters 2 bzw. insbesondere des Kryoballons 3 dar. Um das Risiko zu vermindern, dass es während der Behandlung aufgrund eines Blutstaus vor dem Kryoballon 3 zu einer Unterversorgung der Niere und damit zu einem Niereninfarkt kommt, ist diese Ausführungsform des Kryoballons 3 nicht symmetrisch sondern entspricht der Form eines Zylindersektors. In der hier dargestellten Ausführungsform wird von dem Kryoballon 3 ein Kanal 8 frei gelassen, durch welchen auch im Behandlungsfall Blut in Richtung Niere strömt und diese mit dem nötigen Sauerstoff versorgt.

Der Kryoballon 3 besitzt in dieser Ausführungsform die Gestalt eines Zylindersektors, wobei die Zirkumferenz des Kryoballons einen Radius von mehr als 180°, bevorzugt mehr als 230° aufspannt. Wenn der Zylindersektor einen Radius von mehr als 180° aufspannt, kann gewährleistet werden, dass die gesamte Zirkumferenz der Gefäßwand mit dem Kryoballon 3 in Kontakt kommt, wenn der Kryoballon 3 während der Behandlung wenigstens einmal um die Längsachse um einen Mindestwinkel gedreht wird, welcher größer, als der Winkel ist, welchen der ausgesparte Zylindersektor des Kanals 8 aufspannt.

Vorzugsweise besitzt der Katheter 2 an einem proximalen Ende Markierung(en) 13, mit welchen festgestellt werden kann, ob die während der Behandlung durchgeführte Rotation um die Längsachse größer ist, als der von dem Kanal 8 aufgespannte Winkel. Auf diese Weise kann sichergestellt werden, dass die gesamte Zirkumferenz der renalen Arterie von dem Kryoballon 3 während der Behandlung berührt wird und die renalen perivaskulären Nerven damit auf der gesamten Länge der Arterie und der gesamten Zirkumferenz abladiert werden.

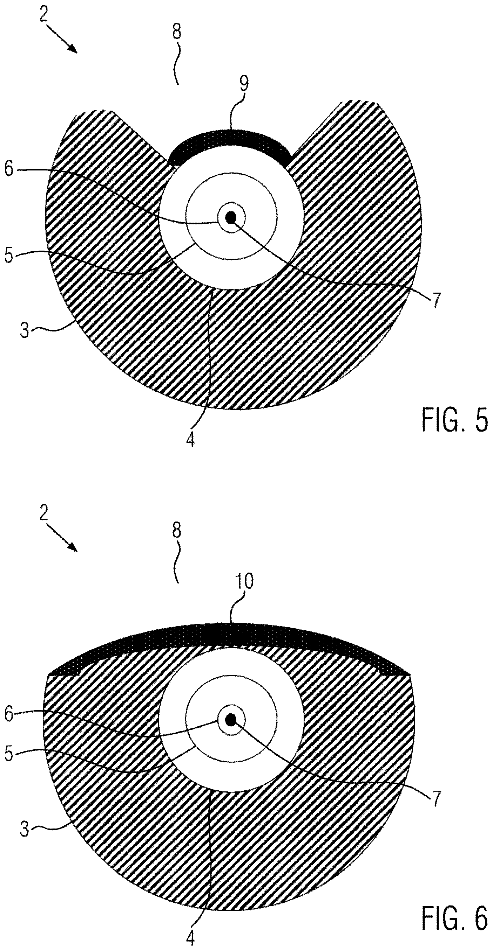

Figur 5 ist eine weitere Ausführungsform, bei der der Kryoballon 3 im Behandlungsfalle nicht die gesamte Innenwand der renalen Arterie berührt und einen Kanal 8 für das Blut freihält. Hierfür ist ein Streifen 9 eines wenig elastischen Materials auf den Katheter 2 vor und hinter dem Kryoballon 3 befestigt, z. B. geklebt. Dieser Streifen 9 verhindert ein Ausdehnen des Kryoballons 3 in dem Bereich des Streifens 9.

Gemäß einer bevorzugten Ausführungsform ist der Streifen 9 aus einem Material mit geringer Wärmeleitfähigkeit gefertigt oder es ist zusätzlich zu dem Streifen 9 ein isolierendes Material vorgesehen, welches verhindert, dass das durch den Kanal 8 strömende Blut im Behandlungsfalle gefriert oder verklumpt.

Figur 6 ist eine weitere Ausführungsform, bei der der Kryoballon 3 im Behandlungsfalle nicht die gesamte Innenwand der renalen Arterie berührt. In diesem Falle ist eine nicht oder wenig dehnbare Schicht 10 vorgesehen, welche auf der Oberfläche des Kryoballons 3 befestigt ist, oder die dehnbare oder gefaltete Oberfläche des Kryoballons 3 durch eine nicht oder wenig dehnbare und/oder nicht entfaltbare Schicht ersetzt. Auch auf diese Art und Weise kann ein Kanal 8 freigehalten werden und es wird während der Behandlung nie die komplette Blutzufuhr der Niere durch den Kryoballon 3 blockiert, sodass das Risiko eines Niereninfarktes deutlich reduziert ist. Wie in der in Figur 5 dargestellten Ausführungsform kann auch bei dieser Ausführung vorgesehen sein, dass die Schicht 10 aus einem Material mit geringer Wärmeleitfähigkeit gebildet ist.

## Patentansprüche

1. Medizinisches Gerät (1) zur Denervierung renaler perivaskulärer Nerven umfassend ein System aus einer Kältemittelquelle (12), einer Steuerung (11) für die Kältemittelquelle (12) sowie einem Katheter (2), der einen flexiblen Schaft zum Einführen in die renale Arterie eines Patienten umfasst, wobei der Schaft ein inneres Lumen zum Zuführen eines Kältemittels und einen am distalen Ende des Schaftes angeordneten Kryoballon (3) aufweist, wobei auf dem Kryoballon (3) wenigstens eine bipolare Elektrode (16) zur Abgabe eines elektrischen Impulses zur Stimulation der perivaskulären Nerven angeordnet ist, **dadurch gekennzeichnet, dass** das medizinische Gerät (1) dazu ausgebildet ist, dass der Kryoballon (3) im Anwendungsfall die Wand der renalen Arterie auf einer Länge von mehr als 2,5 cm durchgängig berührt, durch Einleiten eines Kältemittels kühlt und hierdurch auf einer Länge von mindestens 2,5 cm durchgehend und gleichzeitig über die gesamte Zirkumferenz abladiert, wobei der Katheter über einen Drucksensor zur direkten invasiven Blutdruckmessung während der Denervierung verfügt und im Anwendungsfalle mit dem Sensor die Druckanstiegsgeschwindigkeit delta mmHg/ s misst und die Steuereinheit (11) dazu ausgebildet ist, die Kältemittelquelle bzw. die Zuführung des Kältemittels in den Kryoballon (3) in Abhängigkeit von der Blutdruck-Anstiegsgeschwindigkeit eines Patienten zu steuern.

2. Medizinisches Gerät (1) nach Anspruch 1, **dadurch gekennzeichnet, dass** die Steuereinheit (11) mit der wenigstens einen bipolaren Elektrode (16) zur Abgabe eines elektrischen Impulses zur Stimulation der perivaskulären Nerven verbunden ist, wobei die Steuereinheit (11) bei Feststellung einer Blutdruckerhöhung innerhalb eines vorgegebenen Zeitintervalls um einen vorbestimmten Grenzwert, die auf eine Anregung der perivaskulären Nerven durch die wenigstens eine Elektrode (16) folgt, einen Kältemittelfluss zu dem Kryoballon startet oder aufrecht erhält.

3. Medizinisches Gerät (1) nach Anspruch1, **dadurch gekennzeichnet, dass** die Steuereinheit (11) mit der wenigstens einen Elektrode (16) zur Abgabe eines
elektrischen Impulses zur Anregung der perivaskulären Nerven verbunden ist, wobei die Steuerung bei Feststellung dass eine Blutdruck-Anstiegsgeschwindigkeit unter einem vorbestimmten Grenzwert, als Reaktion auf eine Stimulation der perivaskulären Nerven durch die wenigstens eine Elektrode 16 liegt,
einen Kältemittelfluss zu dem Kryoballon (3) unterbricht.

4. Medizinisches Gerät (1) nach Anspruch 1, **dadurch gekennzeichnet, dass** die Steuereinheit (11) einen Testmodus aufweist, bei dem der Kryoballon (3) auf eine Temperatur gekühlt wird, bei der die Reizleitung der renalen perivaskulären Nervengedämpft oder unterbunden wird, eine Ablation der renalen perivaskulären Nerven jedoch nicht stattfindet.

5. Medizinisches Gerät (1) nach Anspruch 1, **dadurch gekennzeichnet, dass** die Läsionstiefe über eine Impedanzmessung mit Hilfe der Elektroden und der Steuerung durchgeführt wird, wobei die Ablation der renalen perivaskulären Nerven beendet wird, wenn ein vorgegebener Wert für die Ermittlung der Läsionstiefe über die Impedanzmessung ermittelt wurde.

6. Medizinisches Gerät (1) nach Anspruch 1, **dadurch gekennzeichnet, dass** die Behandlung nach einer kumulierten Ablationszeit von 18 Minuten beendet wird.

7. Medizinisches Gerät (1) nach einem der vorangehenden Ansprüche, **dadurch gekennzeichnet, dass** wenigstens 2 Elektroden (16) auf der Außenseite des Kryoballons
(3) angeordnet sind.

8. Medizinisches Gerät (1) nach einem der vorhergehenden Ansprüche mit zusätzlich einem kälteisolierten Kanal (8) für geringen Blutfluss zur Niere während der Ablation.

## Claims

1. A medical device (1) for denervating renal perivascular nerves comprising a system having a refrigerant source (12), a controller (11) for the refrigerant source (12) and a catheter (2) comprising a flexible shaft for insertion into the renal artery of a patient, the shaft having an inner lumen for supplying a refrigerant and a cryoballoon (3) arranged at the distal end of the shaft, wherein at least one bipolar electrode (16) is arranged on the cryoballoon (3) for emitting an electrical impulse for stimulating the perivascular nerves, **characterized in that** the medical device (1) is designed such that, when in use, the cryoballoon (3) touches the wall of the renal artery over an entire length of more than 2.5 cm, cools it by introducing a refrigerant and thereby ablates over an entire length of at least 2.5 cm over the entire circumference, wherein the catheter has a pressure sensor for direct invasive blood pressure measurement during denervation and, in the case of use, measures the rate of pressure rise delta mmHg/s with the sensor, and the control unit (11) is designed to control the source of refrigerant or the supply of refrigerant into the cryoballoon (3) as a function of the rate of rise of a patient's blood pressure.

2. Medical device (1) according to claim 1, **characterized in that** the control unit (11) is connected to the at least one bipolar electrode (16) for emitting of an electrical pulse for stimulating the perivascular nerves, wherein the control unit (11) starts or maintains a flow of refrigerant to the cryoballoon, upon detection of an increase in blood pressure within a predetermined time interval and by a predetermined threshold value in response to a stimulation of the perivascular nerves caused by the at least one electrode (16).

3. Medical device (1) according to claim 1, **characterized in that** the control unit (11) is connected to the at least one electrode (16) for delivering an electrical pulse for stimulating the perivascular nerves, wherein the controller interrupts a flow of refrigerant to the cryoballoon (3) upon detection that a blood pressure rise rate is below a predetermined threshold value in response to a stimulation of the perivascular nerves caused by the at least one electrode (16).

4. Medical device (1) according to claim 1, **characterized in that** the control unit (11) has a test mode in which the cryoballoon (3) is cooled to a temperature at which the stimulation conduction of the renal perivascular nerves is attenuated or interrupted, but ablation of the renal perivascular nerves does not take place.

5. Medical device (1) according to claim 1, **characterized in that** the lesion depth is carried out via an impedance measurement with the aid of the electrodes and the controller whereby the ablation of the renal perivascular nerves is terminated when a predetermined value of the lesion depth has been determined via the impedance measurement.

6. Medical device (1) according to claim 1, **characterized in that** the treatment is terminated after a cumulative ablation time of 18 minutes.

7. Medical device (1) according to one of the preceding claims, **characterized in that** at least 2 electrodes (16) are arranged on the external surface of the cryoballoon (3).

8. Medical device (1) according to one of the preceding claims with additionally a cold-insulated channel (8) for a minimal blood flow to the kidney during ablation.

## Revendications

1. Dispositif médical (1) pour la dénervation des nerfs périvasculaires rénaux comprenant un système ayant une source de réfrigérant (12), un contrôleur (11) pour la source de réfrigérant (12) et un cathéter (2) comprenant une tige flexible pour l'insertion dans l'artère rénale d'un patient, la tige ayant une lumière interne pour l'alimentation en réfrigérant et un cryoballon (3) disposé à l'extrémité distale de la tige, dans lequel au moins une électrode bipolaire (16) est disposée sur le cryoballon (3) pour émettre une impulsion électrique afin de stimuler les nerfs périvasculaires, **caractérisé en ce que** le dispositif médical (1) est conçu de telle sorte que, lorsqu'il est utilisé, le cryoballon (3) touche la paroi de l'artère rénale sur une longueur totale de plus de 2. 5 cm, la refroidisse en introduisant un réfrigérant et effectue ainsi une ablation sur une longueur totale d'au moins 2,5 cm sur toute la circonférence, le cathéter étant doté d'un capteur de pression pour la mesure invasive directe de la pression artérielle pendant la dénervation et, en cas d'utilisation, mesurant la vitesse de montée en pression delta mmHg/s avec le capteur, et l'unité de commande (11) étant conçue pour contrôler la source de réfrigérant ou l'alimentation en réfrigérant dans le cryoballon (3) en fonction de la vitesse de montée de la pression artérielle d'un patient.

2. Dispositif médical (1) selon la revendication 1, **caractérisé en ce que** l'unité de commande (11) est connectée à au moins une électrode bipolaire (16) pour émettre une impulsion électrique afin de stimuler les nerfs périvasculaires, dans lequel l'unité de commande (11) démarre ou maintient un flux de réfrigérant vers le cryoballon, sur détection d'une augmentation de la pression artérielle dans un intervalle de temps prédéterminé et d'une valeur seuil prédéterminée en réponse à une stimulation des nerfs périvasculaires causée par au moins une électrode (16).

3. Dispositif médical (1) selon la revendication 1, **caractérisé en ce que** l'unité de commande (11) est connectée à l'électrode (16) au moins pour délivrer une impulsion électrique afin de stimuler les nerfs périvasculaires, dans lequel le contrôleur interrompt un flux de réfrigérant vers le cryoballon (3) sur détection d'un taux d'augmentation de la pression artérielle inférieur à une valeur seuil prédéterminée en réponse à une stimulation des nerfs périvasculaires causée par l'électrode (16) au moins.

4. Dispositif médical (1) selon la revendication 1, **caractérisé en ce que** l'unité de commande (11) a un mode test dans lequel le cryoballon (3) est refroidi à une température à laquelle la conduction de stimulation des nerfs périvasculaires rénaux est atténuée ou interrompue, mais l'ablation des nerfs périvasculaires rénaux n'a pas lieu.

5. Dispositif médical (1) selon la revendication 1, **caractérisé en ce que** la profondeur de la lésion est mesurée par impédance à l'aide des électrodes et du contrôleur, l'ablation des nerfs périvasculaires rénaux étant interrompue lorsqu'une valeur prédéterminée de la profondeur de la lésion a été déterminée par la mesure de l'impédance.

6. Dispositif médical (1) selon la revendication 1, **caractérisé par le fait que** le traitement est interrompu après une durée d'ablation cumulée de 18 minutes.

7. Dispositif médical (1) selon l'une des revendications précédentes, **caractérisé par le fait qu'**au moins 2 électrodes (16) sont disposées sur la surface externe du cryoballon (3).

8. Dispositif médical (1) selon l'une des revendications précédentes avec en plus un canal isolé du froid (8) pour un flux sanguin minimal vers le rein pendant l'ablation.
